# EUROPEAN PATENT APPLICATION

(11) **EP 3 257 844 A1**
(43) Date of publication of application: **20.12.2017**
(21) Application number: 16174312.5
(22) Date of filing: 14.06.2016
(51) Int. Cl.: C07D 239/20

(54) **METHOD OF PREPARING A HIGH PURITY IMIDAZOLIUM SALT**

(71) Applicant: Evonik Degussa GmbH, 45128 Essen (DE)
(72) Inventor: WILLY, Benjamin, 40211 Düsseldorf (DE); ZEHNACKER, Olivier, 44263 Dortmund (DE); SCHNEIDER, Rolf, 63584 Gründau-Rothenbergen (DE); WANG, Xinming, 242-0003 Kanagawa-ken (JP); ESCHER, Werner, 41462 Neuss (DE); MÜNZNER, Stefan, 46282 Dorsten (DE); HILLER, Christoph, 48249 Dülmen (DE)

(57) **Abstract**

The present invention encompasses a novel method for synthesizing highly pure salts of the general formula Q⁺A⁻, wherein the starting materials are reacted in the presence of water, and wherein Q⁺ is 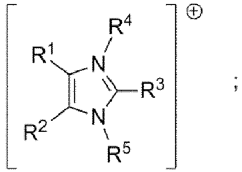 and wherein A⁻ is

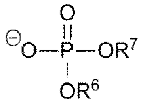

## Description

The invention relates to a method of preparing a high purity imidazolium salt.

### Background of the Invention

In air conditioning systems for the aeration and conditioning of buildings or vehicles, the air generally not only has to be cooled, but also dehumidified since the air to be cooled often has such a high humidity that, upon cooling to the desired temperature, the dew point is fallen below. Hence in conventional air conditioning systems dehumidification of the air accounts for a large part of the electricity consumption.

One option of reducing the electricity consumption of air conditioning systems for buildings is the dehumidification of air by adsorption or absorption of water using a drying medium and a regeneration of the drying medium laden with water by heating to a temperature at which the water is desorbed again. Compared to adsorption on a solid adsorbent, the advantage of absorption in a liquid absorption medium is that drying of air can be performed with reduced equipment complexity and with less drying medium and that regeneration of the water-laden drying medium using solar heat is easier to carry out.

The aqueous solutions of lithium bromide, lithium chloride or calcium chloride hitherto employed as liquid absorption media in commercial air conditioning systems have the disadvantage that they are corrosive towards the metallic materials of construction typically employed in air conditioning systems and that they thus necessitate the use of expensive specific materials of construction. These solutions can additionally cause problems due to salt crystallizing out of the absorption medium.

Ionic liquids comprising dialkylimidazolium ions (as described in WO 2004/016631 A1) have been described as alternatives to lithium salts in the prior art for similar applications.

Y. Luo et al., Appl. Thermal Eng. 31 (2011) 2772-2777 proposes the ionic liquid 1-ethyl-3-methylimidazolium tetrafluoroborate in place of aqueous solutions of lithium bromide for drying of air.

Y. Luo et al., Solar Energy 86 (2012) 2718-2724 proposes the ionic liquid 1,3-dimethyimidazolium acetate as an alternative to 1-ethyl-3-methylimidazolium tetrafluoroborate for drying of air.

US 2011/0247494 A1 proposes, in paragraph [0145], the use of trimethylammonium acetate or 1-ethyl-3-methylimidazolium acetate as liquid drying agent instead of aqueous lithium chloride solution. Example 3 compares water uptake from humid air for a series of further ionic liquids.

However, a problem of ionic liquids comprising dialkylimidazolium ions is that they often comprise impurities, which lead to substances that are odour-intensive or are injurious to health entering the dehumidified air upon a dehumidification of air using the ionic liquid. Moreover, it has been found that during the desorption of water from ionic liquids which contain a basic anion, such as, for example, a carboxylate ion, odour-intensive decomposition products are formed which, in the event of a subsequent use of the ionic liquid for the dehumidification of air, enter the dehumidified air.

In addition, ionic liquids produced by prior art processes often display a yellowish colour, which is undesirable.

Therefore, there remains a need for ionic liquids comprising imidazolium ions, which do not display the disadvantages described above. The problem to be solved by the present invention is hence provision of a process for the production of ionic liquids comprising dialkylimidazolium ions, wherein the level of volatile compounds is brought to a minimum and wherein the ionic liquid is not coloured.

### Detailed Description of the Invention

It has been surprisingly found that the above problem is solved by the process according to the invention, described hereinafter.

The invention hence provides a process for preparing a compound of formula (I): Q⁺A⁻, wherein Q⁺ is 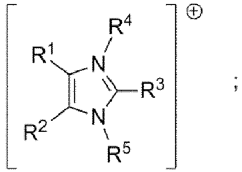 and wherein A⁻ is 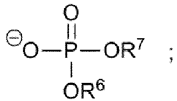 the process comprising reacting a compound of formula **(II)** with a compound of formula **(III)** in
the presence of water, wherein **(II)** and **(III)** are: 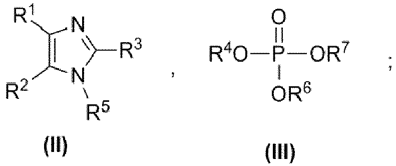 giving the compound of formula **(I),**
wherein:
each of R¹, R², R³ are independently a hydrogen or alkyl, preferably of 1 to 10, more preferably 1 to 8, even more preferably 1 to 6, most preferably 1 to 4 carbon atoms,
each of R⁴, R⁵, R⁶, R⁷ are independently alkyl, preferably of 1 to 10, more preferably 1 to 8, even more preferably 1 to 6, most preferably 1 to 4 carbon atoms.

In a preferred embodiment of the present invention, R¹ = R² = R³ = hydrogen and each of R⁴, R⁵, R⁶, R⁷ are independently methyl or ethyl.

In a more preferred embodiment of the present invention, R¹ = R² = R³ = hydrogen, R⁵ = methyl and each of R⁴, R⁶, R⁷ are independently methyl or ethyl.

In an even more preferred embodiment of the present invention, R¹ = R² = R³ = hydrogen, R⁵ = methyl and R⁴ = R⁶ = R⁷ = ethyl.

In step a) of the process according to the invention, a compound of formula **(II)** is reacted with a compound of formula **(III)** in the presence of water, giving a crude product comprising a compound of formula (I). The skilled person is familiar with the reaction conditions, which are described in WO 2004/016631 A1, for example.

In particular, step a) of the process according to the invention is preferably carried out at a temperature in the range of from 130 °C to 200 °C, more preferably 140 °C to 190 °C, even more preferably 150 °C to 175 °C.

The pressure of the reaction is not critical and may be for example atmospheric pressure, preferably under an inert atmosphere, such as nitrogen.

As the reaction is exothermic, it may be desirable to control the rate of addition in some cases and/or to apply external cooling during the addition step.

In general, the compounds of formula **(II)** and **(III)** are present in stoichiometric amounts, i.e. the molar relation of compound **(II)** to compound **(III)** is in the range 0.9 : 1 to 1.1 : 1, more preferably 1 : 1. In some cases, it might be advantageous to use the imidazole compound **(II)** in a slight excess over the phosphate ester **(III)**, for example in the range of 1.01 to 1.4 molar equivalents, preferable 1.02 to 1.4.

The reaction time is not particularly limited. Typically, the reaction is continued until at least 90 % of the compounds **(II)** or **(III)** has reacted to form compound **(I).** "Completion of the reaction" means that at least 90 % of the compounds **(II)** or **(III)** has reacted to form compound **(I).** The progress of the reaction can be conveniently controlled by methods known to the skilled person, such as NMR.

According to the invention, the reaction is carried out in the presence of water. It has been surprisingly found that even small amounts of water lead to surprising improvements, i.e. bring the level of smelly impurities and the colour number in the resultant product **(I)** to a minimum.

In a preferred embodiment, "presence of water" means that compounds **(II)** and **(III)** are reacted in the presence of at least 1.0 weight-% of water, based on the combined masses of compounds **(II)** and **(III)**. Even more preferred, it means that that compounds **(II)** and **(III)** are reacted in the presence of at least 3.7 weight-% of water, based on the combined masses of compounds **(II)** and **(III)**. Even more preferred, it means that that compounds **(II)** and **(III)** are reacted in the presence of at least 7.4 weight-% of water, based on the combined masses of compounds **(II)** and **(III).** Even more preferred, it means that that compounds **(II)** and **(III)** are reacted in the presence of at least 10.0 weight-% of water, based on the combined masses of compounds **(II)** and **(III).** Even more preferred, it means that that compounds **(II)** and **(III)** are reacted in the presence of at least 20 weight-% of water, based on the combined masses of compounds **(II)** and **(III).** Even more preferred, it means that that compounds **(II)** and **(III)** are reacted in the presence of at least 40 weight-% of water, based on the combined masses of compounds **(II)** and **(III).** Even more preferred, it means that that compounds **(II)** and **(III)** are reacted in the presence of at least 80 weight-% of water, based on the combined masses of compounds **(II)** and **(III).** Even more preferred, it means that that compounds **(II)** and **(III)** are reacted in the presence of at least 100 weight-% of water, based on the combined masses of compounds **(II)** and **(III).** Even more preferred, it means that that compounds **(II)** and **(III)** are reacted in the presence of at least 150 weight-% of water, based on the combined masses of compounds **(II)** and **(III).**

The reaction between compounds **(II)** and **(III)** may be carried out in the presence or absence of an organic solvent, while it is preferred to carry it out in the absence of an organic solvent.

"Organic solvent" means organic compounds which are known to the skilled person as solvents such as (and preferably) selected from the group consisting of aliphatic solvents, preferably pentane, hexane, heptane, octane, decane, cyclohexane, tetramethylsilane; aromatic solvents, preferably benzene, toluene, xylene; ether compounds, preferably diethyl ether, dipropyl ether, dibutyl ether, methyl *tert*-butyl ether; halogenated solvents, preferably dichloromethane, chloroform, tetrachloromethane; alcohols, preferably methanol, ethanol, propanol, *iso*-propanol, butanol, *tert*-butanol; esters, preferably methyl acetate, ethyl acetate, propyl acetate, butyl acetate; acetone. Polar organic solvents such as esters and alcohols are particularly preferred.

"Absence of an organic solvent" means particularly that the overall content of all organic solvents in the reaction mixture is below 10 weight-% based on the sum of the weights of compounds **(II)** and **(III)**, preferably below 5 weight-% b based on the sum of the weights of compounds **(II)** and **(III)**, more preferably below 1 weight-% based on the sum of the weights of compounds **(II)** and **(III).**

After completion of the reaction, the water (and the organic solvent, in case the reaction is carried out in the presence of such organic solvent), can be at least partially removed by the methods described in the prior art.

Such at least partial removal can be carried out by extraction, stripping, distillation or any other process known to the skilled person, preferably by extraction, stripping, distillation.

In this context, "partial removal" means in particular, that at least 50 % of the water (or respectively of the water and the organic solvent, in case the reaction is carried out in the presence of such organic solvent) in the reaction mixture is removed, preferably at least 70 %, even more preferably at least 90 %, even more preferably 99 % of the water (or in each case the respective amount of the water and the organic solvent, in case the reaction is carried out in the presence of such organic solvent) is removed.

For carrying out the distillation, all apparatuses known to the person skilled in the art can be used, thus e.g. a stirred reactor, a falling-film evaporator or a thin-film evaporator, in each case in combination with a suitable distillation column or another apparatus suitable for the distillation.

The method of the present invention thus provides as a product an imidazolium salt with a surprisingly low APHA number (indicative of a low level of discolouring) and a low level of odorous impurities such as amines and *N*-methylimidazole.

The following examples illustrate the invention.

### Examples

### Materials

In the following examples, *N*-methylimidazole (CAS number: 616-47-7) and triethylphosphate (CAS number: 78-40-0) were purchased from Sigma Aldrich.

### Methods

The APHA numbers were determined by diluting the respective sample 1 : 1 with water and determining the number according to the procedure described in DIN EN ISO 6271 (2005).

The impurities were determined by headspace GC/MS as follows: 0.1 g of the sample were incubated for 20 minutes at 70 °C in a sampler. The composition of the gas phase was analyzed by condensating it in a cooling trap and analyzing the condensate with gas chromatography ("GC") and mass spectrometry ("MS"). GC was performed with an apparatus of Hewlett Packard ("HP 6890"; sampler: Turbomatrix 40, Perkin Elmer). MS was performed with an apparatus of Hewlett-Packard ("HP 5973").

### Comparative Examples V1, V2:

Triethylphosphate (929 g, 5.0 mole) was added dropwise over 2 hours to a reaction vessel containing *N* methylimidazole (411 g, 5.0 mole). Afterwards, the reaction mixture was heated up to 130 °C and stirred under reflux for 14 h. Then, the volatile parts were removed under reduced pressure with a rotary evaporator. 1-Ethyl-3-methylimidazole diethylphosphate was obtained in 98 % **(V1)** and 99 % **(V2)** yield, respectively.

### Inventive examples E1 and E2:

Triethylphosphate (929 g, 5.0 mole) was added dropwise over 2 hours to a reaction vessel containing *N*-methylimidazole (411 g, 5.0 mole) and water (**E1:** 50 mL; **E2:** 100 mL). Afterwards the reaction mixture was heated up to 130 °C and stirred under reflux for 14 h. Then, the volatile parts were removed under reduced pressure with a rotary evaporator. In each case, 1-ethyl-3-methylimidazole diethylphosphate was obtained in 99 % yield.

Then the residuals in each sample were assessed with GC/MS analysis as described above. The numbers given in the table for each impurity represent the observed peak height, which, in turn, is proportional to the content of each impurity. Moreover, the APHA number was determined by the method described above.

| Ex. | Water added [ml] | Impurities detected by GC/MS | | | | APHA number |
|---|---|---|---|---|---|---|
| | | *iso*-propanol | ethyl amine | | triethyl amine *N*-methylimidazole | |
| **V1** | 0 | 80 | 60 | 40 | 520 | 905 |
| **V2** | 0 | 80 | 140 | 30 | 760 | 803 |
| **E1** | 50 | 0 | 0 | 10 | 200 | 47 |
| **E2** | 100 | 0 | 0 | 10 | 190 | 32 |

The results summarized in the above table show that the process according to the invention, i.e. carrying out the reaction in the presence of water, lead to surprisingly pure products in terms of colourlessness (the APHA number is reduced by over ~95 %) and in terms of a reduction of impurities in the product. These results were surprising.

## Claims

1. A process for preparing a compound of formula (I): Q⁺A⁻, wherein Q⁺ is 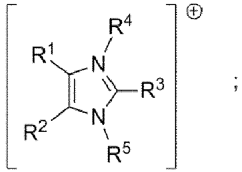 and wherein A⁻ is 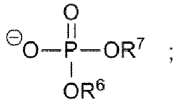 the process comprising reacting a compound of formula **(II)** with a compound of formula **(III)** in the presence of water, wherein **(II)** and **(III)** are: 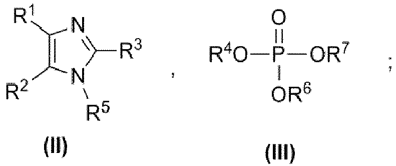 giving the compound of formula (I),
wherein:
each of R¹, R², R³ are independently a hydrogen or alkyl,
each of R⁴, R⁵, R⁶, R⁷ are independently alkyl.

2. A process according to claim 1, wherein each of R¹, R², R³ are independently a hydrogen or alkyl of 1 to 10 carbon atoms, and each of R⁴, R⁵, R⁶, R⁷ are independently alkyl of 1 to 10 carbon atoms.

3. A process according to claim 2, wherein R¹ = R² = R³ = hydrogen and wherein each of R⁴, R⁵, R⁶, R⁷ are independently methyl or ethyl.

4. A process according to claim 3, wherein R¹ = R² = R³ = hydrogen, R⁵ = methyl and wherein each of R⁴, R⁶, R⁷ are independently methyl or ethyl.

5. A process according to claim 4, wherein R¹ = R² = R³ = hydrogen and R⁵ = methyl and R⁴ = R⁶ = R⁷ = ethyl.

6. A process according to claims 1 to 5, wherein compounds **(II)** and **(III)** are reacted in the presence of at least 1.0 weight-% of water, based on the combined masses of compounds **(II)** and **(III)**.

7. A process according to one of claims 1 to 6, which is carried out in the absence of an organic solvent.

8. A process according to one of claims 1 to 6, which is carried out in the presence of an organic solvent.

9. A process according to one of claims 1 to 8, wherein after completion of the reaction, water and, in case the reaction is carried out in the presence of an organic solvent, the organic solvent are at least partially removed.

10. A process according to one of claims 1 to 9, wherein the reaction is carried out at a temperature of 130 °C to 200 °C.
